# EUROPEAN PATENT SPECIFICATION

(11) **EP 2 452 697 B1**
(45) Date of publication and mention of the grant of the patent: **02.03.2016**
(21) Application number: 11188687.5
(22) Date of filing: 10.11.2011
(51) Int. Cl.: A61L 9/012, A61L 9/013

(54) **Composition to eliminate unpleasant smells**
Zusammensetzung für die Eliminierung von üblen Gerüchen
Composition pour éliminer les odeurs désagréables

(30) Priority: 11.11.2010 IT UD20100204
(43) Date of publication of application: 16.05.2012
(73) Proprietor: Universita' Degli Studi di Udine, 33100 Udine (IT)
(72) Inventor: Bertoni, Aldo, 33010 Reana del Rojale (UD) (IT); Contin, Marco, 33100 Udine (IT); Cudini, Andrea, 33030 Coseano (UD) (IT); De Nobili, Maria, 33033 Codroipo (UD) (IT)
(74) Representative: Petraz, Gilberto Luigi

(56) References cited:
- EP-A1- 1 157 672
- WO-A1-03/060003
- WO-A2-00/32041
- JP-A- 3 182 259
- JP-A- 2008 094 989
- US-A1- 2004 146 855
- US-A1- 2009 223 892

## Description

### FIELD OF THE INVENTION

The present invention concerns a composition to eliminate unpleasant smells, in particular in the form of a dry powder that can be sprinkled and is distributed on organic waste, mainly to prevent unpleasant smells from developing.

### BACKGROUND OF THE INVENTION

It is known that waste disposal is a serious problem from an economic and also environmental point of view for local administrations. There is a tendency to encourage the differentiated collection of solid urban waste (SUW) to promote the recycling of materials and to reduce rubbish sent to the pit. Therefore, apart from collecting glass, plastic and paper, the strategy is also adopted to collect the separated organic fraction of the SUW, the so-called "organic" and "dry non-recyclable", which also comprises materials contaminated by putrescible substances and therefore potential sources of unpleasant smells (diapers, oily papers etc.). These fractions are collected door-to-door on pre-established days, or delivered by the users themselves in suitable bins. One disadvantage of storing, transporting and processing humid or dry non-recyclable organic waste is the rapid development of unpleasant smells of various type. This happens with greater intensity during the hotter months. This problem is particularly felt by those who are obliged to keep the humid or dry non-recyclable waste in his/her house for several days, waiting for the day when the waste is collected by the operators.

The problem of smells has been faced in the management of composting plants and in processing urban or zootechnical sewage with plant-engineering techniques, intervening on the smells when they have already formed, not on the causes thereof, aiming to absorb them before they spread into the environment.

Known techniques of this kind include captation and purification using biofilters, but these entail high investment and management costs and in any case do not guarantee total elimination of the malodorous substances.

In the domestic environment, the solutions proposed are specific deodorant products that reduce the perception or cover the smells after they have been emitted into the air, with the emission of perfumed substances.

Chemical additives have been proposed, such as iron oxides, manganese oxides and nitrates, to reduce the malodorous emissions during the processing of sludge and sewage, while additives of a biological nature, enzymes and microorganisms are also used, but these have little practical success in the field of domestic composting or the small-scale production of compost.

A process is known, from US-A-4,108,771, to reduce the emanation of smells from waste, which provides to use acid solutions containing an oxidant agent chosen from ammonium nitrates, chlorates and permanganates and metal alkalis, and a precipitating agent chosen from water-soluble ferric or ferrous compounds.

Document US-A-5,984,993 proposes to control the smells from waste by means of chlorate salts, of sodium, calcium or potassium, and nitrate salts, typically sodium nitrate.

Document US-A-2002/102229 describes the combination of oxides that eliminate existing smells, and nitrates that prevent others from forming.

Document WO-A-97/06834 describes a deodorant in granules with a nitrate base, especially of magnesium, sodium, ammonium, calcium and/or potassium, combined with a fragrance and a drying agent, typically silica or silicates.

Document WO-A-2004080562 discloses a process to remove the smell of hydrogen sulfide and volatile substances from reduced sulfur deriving from waste, treating them with ferric nitrate at a ratio of less than 2.4 parts of nitrate to one part of reduced sulfur.

The chemical additives cited have scarcely been used in practice, due to their limited effectiveness and for problems connected to the hygroscopicity of the preparations in granule or powder form which tend to solidify and harden. On the other hand, liquid preparations are not suitable for use with the humid fraction because they promote the formation of percolates and dripping.

In the domestic field there are no products currently on the market that allow to intervene directly and easily on the organic waste stored in the home rubbish bin in order to prevent the formation of smells. Domestic solutions, as we said, are the deodorant type that cover the smells by spreading fragrances.

Document US-A-2004/146855 (US'855) describes the preparation of supermagnetic particles containing iron that can be used in the identification and purification of biological materials and are prepared starting from, for example, a chitosan-Fe(II) complex.

Document WO-A-03/060003 (WO'003) describes the production of solid, hard surfaces or plates with anti-microbe properties. The surfaces incorporate in their matrix anti-microbe agents represented by complexes of chitosan and metals, in particular copper, zinc or silver. In particular, one problem with using silver is its high cost as raw material, which, considering the application and the purposes in question, in practice prevents it from being used in large quantities, for economic reasons.

Document JP-A-3182259 discloses a deodorizing paper made using a complex powder in which a salt of a metal selected from transition elements of the 4th period of the periodic table forms a complex with chitosan, preferably, in the ratio of 0.3-2.3 per unit of amino glucosamine repeat unit in the chitosan branched chain.

Document US-A-2009/223892 discloses the use of nitrate salts for suppressing the development of biological odors in the sanitary field, in particular in waste-pipes, drains, waste traps, pump units and/or in waste-water-carrying areas.

Purpose of the present invention is to allow the formulation of compositions in powder form which prevent the development of malodorous volatile substances from solid urban waste (SUW) and their fractions, collected separately, such as specifically the humid (organic) part and the so-called non-recyclable dry part, both in the domestic field, and also for treating bins, means of transport and storage containers in general by collection operators, and also for the processing of SUW and agricultural food industrial waste, the green fraction deriving from public and private gardens, sludge from purification and sewage.

The Applicant has devised, tested and embodied the present invention to overcome the shortcomings of the state of the art and to obtain these and other purposes and advantages.

Unless otherwise defined, all the technical and scientific terms used here and hereafter have the same meaning as commonly understood by a person with ordinary experience in the field of the art to which the present invention belongs.

Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively. The percentage content in weight of the various components of the present invention is intended as referring to the weight of the overall composition.

### SUMMARY OF THE INVENTION

The present invention is set forth and characterized in the independent claim, while the dependent claims describe other characteristics of the invention or variants to the main inventive idea.

In accordance with the above purpose, a composition in the form of a dry sprinklable powder according to the present invention is used by distributing and sprinkling it to prevent the development of malodorous volatile substances from solid urban waste (SUW) and their fractions collected separately, such as the humid fraction and non-recyclable dry waste.

The composition according to the present invention comprises chitosan or chitosan nitrate and one or more nitrogen-based electron-acceptor components and one or more electron-acceptor components based on iron (III) and/or manganese, with the exclusion of components comprising silver and of chitosan-metal complexes.

Chitosan, the common name of poly[1-4]-β-N-acetyl-D-glucosamine, is a natural organic polycation, therefore with a positive charge, whereas nitrate is an anion, and therefore with a negative charge. Chitosan derives from chitin, a substance contained in fungi and in the exoskeletons of insects and crustaceans. Industrial preparation generally uses shrimp and prawn shells, which are a waste product of the frozen food industry. Consequently, it is available industrially in large quantities and at limited cost. Furthermore, it is neither toxic nor dangerous for the environment, and is normally used in the formulation of dietary integrators due to its ability to bond fats in the intestine and make them less assimilable.

The prevention and elimination of the development of smells is obtained by a synergic effect between chitosan and nitrate.

The synergic effect is due to the affinity of chitosan with malodorous aliphatic compounds, typically volatile acids, which are thus absorbed and retained by the chitosan, and the action of the chitosan nitrate which keeps the nitrate available, preventing extreme anaerobiosis. Furthermore, chitosan has a naturally anti-bacterial action.

The synergy is based particularly on an adjustment of the potential oxidation-reduction given by the electron-acceptor component, advantageously a nitrate, which controls and limits the extreme anaerobiosis of the microorganisms present from which part of the unpleasant smells originate.

For example, chitosan nitrate not only abates the smells connected to the volatile fatty acids, but also prevents the formation of hydrogen sulfide and volatile substances with reduced sulfur that contribute to the formation of unpleasant smells.

Chitosan nitrate can be obtained by reaction, direct or in solution, between chitosan and nitrate, or by the action of suitable microorganisms on ammonium salts.

If chitosan is present in the composition, the presence of nitrate is necessary to obtain the formation of chitosan nitrate in the final composition and to stabilize the compound with regard to the exchange with other anions.

The electron-acceptor components are chosen from one or more inorganic nitrate compounds, therefore different from chitosan nitrate, salts of iron (III) or manganese or oxides of iron (III) or manganese oxides.

In some embodiments, the chitosan, also in nitrate form, is present in the composition in a range from about 0.5% to about 30% in weight.

In this solution, the chitosan nitrate performs both the function of anti-bacterial and affinity with the malodorous compounds, given by the chitosan, and also the function of electron-acceptor, given by the nitrate, and therefore does not need other electron-acceptor components, even if the latter could in any case be present in some embodiments.

The inorganic nitrate compound comprises one or more compounds chosen from a group comprising potassium nitrate, ammonium nitrate, calcium nitrate, magnesium nitrate or iron (III) nitrate.

In some embodiments, the inorganic nitrate compound is present in the composition in a range from about 50% to about 90% in weight.

The initial iron-based compounds are salts of iron (III) or oxides of iron (III), and not of iron (II) as described in US'855. Furthermore, unlike US'855, the percentage composition of nitrate provided in some embodiments of the invention, between about 50% and about 90% in weight, is much higher than that used in US'855.

Furthermore, in some embodiments the present invention contains both chitosan nitrate and metals but, unlike WO'003, does not provide the addition of chitosan-metal complexes. Moreover the present invention does not contain the metals cited in WO'003 as components of the anti-microbe agent, that is, silver, copper and zinc. Furthermore, in some embodiments of the present invention, the nitrate is also present as chitosan nitrate, which is a salt that does not contain silver, whereas in WO'003 the nitrate is present only as a chitosan-silver nitrate complex, which is a complex that does contain silver. Finally, compared with WO'003, the present invention is in powder form, and not in solid form.

The advantageous technical effect of the inorganic nitrate compound is that it functions as a reserve of counter-ions for the chitosan, saturating it. Moreover, since it is an electron-acceptor, it promotes the proliferation of optional anaerobic microorganisms at the expense of obligatory anaerobes, and prevents the onset of fermentation processes.

In some embodiments, the salt of iron (III) or manganese or the oxide of iron (III) or manganese is present in the composition in a range from about 1% to about 15% in weight.

The salt of iron (III) or manganese or the oxide of iron (III) or manganese too, being an electron-acceptor, promotes the proliferation of optional anaerobic microorganisms at the expense of the obligatory anaerobes and prevents the onset of fermentation processes.

In some embodiments, the composition according to the present invention may comprise ferric ammonium sulfate, possibly in a range from about 1% to about 15% in weight.

The ferric ammonium sulfate has the advantageous technical effect of improving the availability of iron (III), and does not constitute a source of nitrogen or nitrogen-based ions for processing.

In some embodiments, the composition according to the present invention may comprise other oxides, as well as the iron (III) oxides, for example carbonates. In one embodiment, the composition according to the present invention comprises calcium carbonate. In variants of this embodiment, the calcium carbonate is present in the composition in a range from about 1% to about 21 % in weight. The advantageous technical effect of the oxides as above, in particular calcium carbonate, is that they have a buffer function on the pH, they contribute to the dehydration of the waste and reduce the volatility of the fatty acids, promoting their absorption on the chitosan.

In other embodiments, the composition according to the present invention may comprise one or more clay-based components, such as bentonite. In variants of this embodiment, the one or more clay-based components are between about 1 % and about 25% in weight. The clay-based components stabilize the chitosan that is absorbed upon them, and promote the dehydration of the waste.

In other embodiments, the composition according to the present invention may comprise one or more components based on humic acids, such as leonardite. In variants of this embodiment, the one or more components based on humic acids are present in a percentage of less than about 24% in weight. Leonardite has the advantageous effect of promoting the transfer of electrons toward the acceptors, such as iron (III) or nitrates.

In other embodiments, the composition according to the present invention may comprise one or more components with an insecticide action, such as *Bacillus thuringiensis* var. *israelensis* (Bti), which is a microorganism with an insecticide action. In variants of this embodiment, the one or more components with an insecticide action are between about 0.1% and about 5% in weight.

The advantageous technical effect of the Bti insecticide action is that it kills larva, particularly of the Diptera, and prevents flies and larvae etc. from reproducing, improving the hygienic aspects of storing domestic waste.

The composition according to the present invention is a dry, sprinklable powder, stable over time and easy to keep. It is also easy to use, to prevent the development of unpleasant odors of various types which usually emanate from organic waste during storage, transport or processing. The present invention has an opposite approach to the problem of smells compared to the state of the art, which simply covers them, since the present invention prevents them from developing and is therefore very effective.

Furthermore, the composition according to the present invention that eliminates smells is economical, since the costs of its components are extremely limited, guaranteeing an economical and environmentally-friendly processing. Indeed, the composition according to the present invention is not toxic and not dangerous for the environment; it also allows an effective preventative action on the development of unpleasant smells.

The composition according to the present invention is in dry powder form, able to be sprinkled, and can therefore easily be distributed and sprinkled on the waste in the container in which the waste is stored, by means of a holed dosing device, advantageously made of recyclable material.

## Claims

1. Composition in dried powder form, able to be sprinkled for use by distribution and sprinkling, and effective for preventing the development of evil-smelling volatile substances from solid urban waste and their fractions collected separately, comprising chitosan or chitosan nitrate **characterized in that** it comprises one or more inorganic nitrate electron-acceptor components chosen from a group consisting of potassium nitrate, ammonium nitrate, calcium nitrate, magnesium nitrate or nitrate of iron (III) and one or more further salts of iron (III) or salts of manganese or oxides of iron (III) or oxides of manganese electron-acceptor components, with the exclusion of components comprising silver and of chitosan-metal complexes.

2. Composition as in claim 1, **characterized in that** the chitosan, possibly also in the form of chitosan nitrate, is present in the composition within a range from between about 0.5% to about 30% in weight.

3. Composition as in any claim hereinbefore, **characterized in that** the inorganic nitrate compound is present in the composition within a range from between about 50% to about 90% in weight.

4. Composition as in any claim hereinbefore, **characterized in that** the salt of iron (III) or manganese or oxide of iron (III) or manganese is present in the composition within a range between about 1% to about 15% in weight.

5. Composition as in any claim hereinbefore, **characterized in that** it further comprises ammonium ferric sulfate, possibly within a range from about 1% to about 15% in weight.

6. Composition as in any claim hereinbefore, **characterized in that** it comprises other oxides, as well as the oxides of iron (III).

7. Composition as in claim 6, **characterized in that** it comprises carbonates, particularly calcium carbonate.

8. Composition as in claim 7, **characterized in that** the calcium carbonate is present in the composition within a range from about 1% to about 21% in weight.

9. Composition as in any claim hereinbefore, **characterized in that** it comprises one or more clay-based components, such as bentonite.

10. Composition as in claim 9, **characterized in that** the one or more clay-based components are between about 1% and about 25% in weight.

11. Composition as in any claim hereinbefore, **characterized in that** it comprises one or more components based on humic acids, such as leonardite.

12. Composition as in claim 11, **characterized in that** the one or more components based on humic acids are present to a percentage lower than 24% in weight.

13. Composition as in any claim hereinbefore, **characterized in that** it comprises one or more components with an insecticide action, wherein in particular said component can be *Bacillus thuringiensis* var. *israelensis* (Bti).

14. Composition as in claim 13, **characterized in that** the one or more components with an insecticide action are between 0.1% and 5% in weight.

## Patentansprüche

1. Zusammensetzung in Trockenpulverform, die zur Verwendung durch Verteilung und Bestreuen gestreut werden kann und wirksam ist, das Entstehen von übelriechenden flüchtigen Stoffen aus festem Haushaltsmüll und aus dessen getrennt gesammelten Anteilen zu verhindern, umfassend Chitosan oder Chitosannitrat, **dadurch gekennzeichnet, dass** sie einen oder mehrere Elektronenakzeptor-Bestandteile anorganische Nitrate umfasst, die aus einer Gruppe gewählt sind, die aus Kaliumnitrat, Ammoniumnitrat, Calciumnitrat, Magnesiumnitrat oder Eisen(III)-nitrat besteht, und ein oder mehrere zusätzliche Elektronenakzeptor-Bestandteile Eisen(III)-salze oder Mangansalze oder Eisen(III)-oxide oder Manganoxide umfasst, mit der Ausnahme von Bestandteilen, die Silber umfassen, und von Chitosan-Metall-Komplexen.

2. Zusammensetzung nach Anspruch 1, **dadurch gekennzeichnet, dass** das Chitosan, möglicherweise auch in Form von Chitosannitrat, in der Zusammensetzung in einem Bereich zwischen ungefähr 0,5 Gew.-% und ungefähr 30 Gew.-% vorhanden ist.

3. Zusammensetzung nach einem der vorhergehenden Ansprüche, **dadurch gekennzeichnet, dass** die anorganische Nitratverbindung in der Zusammensetzung in einem Bereich zwischen ungefähr 50 Gew.-% und ungefähr 90 Gew.-% vorhanden ist.

4. Zusammensetzung nach einem der vorhergehenden Ansprüche, **dadurch gekennzeichnet, dass** das Eisen(III)-salz oder Mangansalz oder Eisen(III)-oxid oder Manganoxid in der Zusammensetzung in einem Bereich zwischen ungefähr 1 Gew.-% und ungefähr 15 Gew.-% vorhanden ist.

5. Zusammensetzung nach einem der vorhergehenden Ansprüche, **dadurch gekennzeichnet, dass** sie ferner Ammoniumeisensulfat, möglicherweise in einem Bereich zwischen ungefähr 1 Gew.-% und ungefähr 15 Gew.-%, umfasst.

6. Zusammensetzung nach einem der vorhergehenden Ansprüche, **dadurch gekennzeichnet, dass** sie andere Oxide, sowie die Eisen(III)-oxide, umfasst.

7. Zusammensetzung nach Anspruch 6, **dadurch gekennzeichnet, dass** sie Carbonate, insbesondere Calciumcarbonat umfasst.

8. Zusammensetzung nach Anspruch 7, **dadurch gekennzeichnet, dass** das Calciumcarbonat in der Zusammensetzung in einem Bereich zwischen ungefähr 1 Gew.-% und ungefähr 21 Gew.-% vorhanden ist.

9. Zusammensetzung nach einem der vorhergehenden Ansprüche, **dadurch gekennzeichnet, dass** sie einen oder mehrere Bestandteile auf Tonbasis, wie Bentonit, umfasst.

10. Zusammensetzung nach Anspruch 9, **dadurch gekennzeichnet, dass** der eine oder die mehreren Bestandteile auf Tonbasis zwischen ungefähr 1 Ges.-% und ungefähr 25 Gew.-% vorliegen.

11. Zusammensetzung nach einem der vorhergehenden Ansprüche, **dadurch gekennzeichnet, dass** sie einen oder mehrere Bestandteile auf Huminsäurebasis, wie Leonardit, umfasst.

12. Zusammensetzung nach Anspruch 11, **dadurch gekennzeichnet, dass** der eine oder die mehreren Bestandteile auf Huminsäurebasis in einem Prozentgehalt niedriger als 24 Gew.-% vorliegen.

13. Zusammensetzung nach einem der vorhergehenden Ansprüche, **dadurch gekennzeichnet, dass** sie einen oder mehrere Bestandteile mit einer insektiziden Wirkung umfasst, worin insbesondere der genannte Bestandteil *Bacillus thuringiensis* var. *israelensis* (Bti) sein kann.

14. Zusammensetzung nach Anspruch 13, **dadurch gekennzeichnet, dass** der eine oder die mehreren Bestandteile mit einer insektiziden Wirkung zwischen 0,1 Gew.-% und 5 Gew.-% vorliegen.

## Revendications

1. Composition sous forme de poudre sèche, apte à être pulvérisée pour une utilisation par distribution et pulvérisation, et efficace dans la prévention du développement de substances volatiles malodorantes émanant de déchets urbains solides et de leurs fractions collectées séparément, comprenant du chitosane ou du citrate de chitosane, **caractérisée en ce qu**'elle comprend un ou plusieurs composant(s) accepteur(s) d'électrons à base de nitrate minéral, choisi(s) dans le groupe constitué par les composants accepteurs d'électrons à base de nitrate de potassium, de nitrate d'ammonium, de nitrate de calcium, de nitrate de magnésium ou de nitrate de fer (III) et d'un ou plusieurs autre(s) sel(s) de fer (III) ou sel(s) de manganèse ou d'oxydes de fer (III) ou d'oxydes de manganèse, à l'exclusion des composants comprenant de l'argent et des complexes chitosane-métal.

2. Composition selon la revendication 1, **caractérisée en ce que** le chitosane, éventuellement également sous forme de nitrate de chitosane, est présent dans la composition à raison d'environ 0,5% à environ 30% en poids.

3. Composition selon l'une quelconque des revendications précédentes, **caractérisée en ce que** le nitrate minéral est présent dans la composition à raison d'environ 50% à environ 90% en poids.

4. Composition selon l'une quelconque des revendications précédentes, **caractérisée en ce que** le sel de fer (III) ou de manganèse ou l'oxyde de fer (III) ou de manganèse est présent dans la composition à raison d'environ 1% à environ 15% en poids.

5. Composition selon l'une quelconque des revendications précédentes, **caractérisée en ce qu**'elle comprend en outre du sulfate d'ammonium ferrique, éventuellement à raison d'environ 1% à environ 15% en poids.

6. Composition selon l'une quelconque des revendications précédentes, **caractérisée en ce qu**'elle comprend d'autres oxydes, ainsi que des oxydes de fer (III).

7. Composition selon la revendication 6, **caractérisée en ce qu**'elle comprend des carbonates, en particulier du carbonate de calcium.

8. Composition selon la revendication 7, **caractérisée en ce que** le carbonate de calcium est présent dans la composition à raison d'environ 1% à environ 21% en poids.

9. Composition selon l'une quelconque des revendications précédentes, **caractérisée en ce qu'**elle comprend un ou plusieurs composant(s) à base d'argile, tels que la bentonite.

10. Composition selon la revendication 9, **caractérisée en ce que** le ou les composant(s) à base d'argile est/sont présent(s) à raison d'environ 1 à environ 25% en poids.

11. Composition selon l'une quelconque des revendications précédentes, **caractérisée en ce qu**'elle comprend un ou plusieurs composant(s) à base d'acides humiques, tels que la léonardite.

12. Composition selon la revendication 11, **caractérisée en ce que** le ou les composant(s) à base d'acides humiques est/sont présents à raison de moins de 24% en poids.

13. Composition selon l'une quelconque des revendications précédentes, **caractérisée en ce qu**'elle comprend un ou plusieurs composant(s) ayant une action insecticide, ledit composant pouvant en particulier être le *Bacillus thuringiensis,* variété *israelensis* (Bti).

14. Composition selon la revendication 13, **caractérisée en ce que** le ou les composant(s) ayant une action insecticide est/sont présent(s) à raison de 0,1 à 5% en poids.
